# FASCICULE DE BREVET EUROPEEN

(11) **EP 2 913 327 B1**
(45) Date de publication et mention de la délivrance du brevet: **07.06.2023**
(21) Numéro de dépôt: 15159302.7
(22) Date de dépôt: 12.07.2007
(51) Int. Cl.: C07D 279/20, A61P 31/00, A61P 31/04, A61P 25/18, A61P 25/24, A61P 25/28, A61P 31/12, A61P 31/18, A61P 33/06, A61P 7/00, A61P 25/00, A61P 31/14, A61P 35/00

(54) **Bleu de méthylène et son utilisation médicale**
Methylenblau und seine Verwendung als Arzneimittel
Methylene blue and its medical use

(30) Priorité: 12.07.2006 FR 0606330
(43) Date de publication de la demande: 02.09.2015
(62) Demande divisionnaire de: 07803863.5
(73) Titulaire: Provepharm Life Solutions, 13013 Marseille (FR)
(72) Inventeur: Feraud, M. Michel, 13013 Marseille (FR); Sayah, M. Babak, 13013 Marseille (FR)
(74) Mandataire: Corizzi, Valérie

(56) Documents cités:
- WO-A-2005/054217
- WO-A2-2006/032879
- WO-A2-2008/007074
- FR-A1- 2 810 318

## Description

La présente invention décrit un nouveau procédé de préparation de composés du type diaminophénothiazinium, en particulier un procédé de purification de ces composés. Elle concerne en particulier le bleu de méthylène, et elle a pour objet le bleu de méthylène résultant de ce procédé dont le degré de pureté est plus élevé que ceux connus de l'art antérieur. Elle a encore pour objet l'utilisation de ce bleu de méthylène pour la préparation de médicaments.

Le chlorure de méthylthioninium, également connu sous les noms de bleu de méthylène ou chlorure de 3,7-bis(diméthylamino)phénothiazine-5-ylium, est un composé organique répondant à la formule ci-dessous :

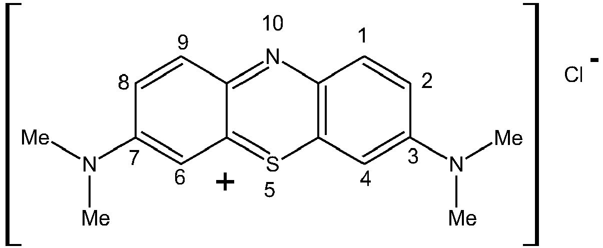

Ce composé a été utilisé de longue date comme colorant et indicateur redox, comme révélateur optique dans des systèmes biophysiques, dans des matériaux nanoporeux comme matériau séparateur, et en imagerie photoélectrochromique. Il est également connu pour ses applications comme agent antiseptique, antiinfectieux, comme antidote et comme agent de diagnostique. Il trouve des utilisations notamment en gynécologie, néonatalogie, cancérologie, oncologie, urologie, ophtalmologie et gastro-entérologie. De nouvelles utilisations dans le domaine thérapeutique sont en cours de développement, telle que la réduction des contaminants pathogéniques dans le sang (GB2373787), la prévention ou l'inhibition d'une réaction hémodynamique excessive (WO03/082296).

De nombreuses méthodes de synthèse ont été décrites pour ce composé, depuis la plus ancienne en 1877 (Brevet allemand n° 1886). Toutes ces méthodes ont en commun d'utiliser des composés métalliques dans au moins une étape de synthèse :
Le Brevet DE-1886 décrit un procédé dans lequel on fait un couplage oxydant du N,N-diméthyl-1,4-diaminobenzène avec H₂S et FeCl₃.

Fiez David et al., « Fundamental Processes of Dye Chemistry », 1949, Interscience, 308-314, décrit un procédé dans lequel le cycle thiazine est formé par traitement au dioxyde de manganèse ou au sulfate de cuivre. Ce procédé comporte encore un traitement au chlorure de zinc et au dichromate de sodium et au thiosulfate d'aluminium.

Le document WO 2005/054217 décrit des dérivés du bleu de méthylène et un procédé pour leur préparation. La méthode de préparation de ces composés utilise la phénotiazine comme produit de départ. Or toutes les méthodes de préparation connues de la phénotiazine font appel à des réactifs métalliques dont les atomes de métaux viennent chélater la phénotiazine à la fin de la synthèse. Les produits obtenus par ce procédé sont donc naturellement contaminés par des résidus métalliques, outre les contaminants organiques habituels comme l'azur B.

Le document WO 2006/032879 décrit un procédé de préparation du bleu de méthylène qui comporte une étape de réduction par du fer, une étape d'oxydation par du dichromate de sodium, une étape d'oxydation par du sulfate de cuivre.

Ces procédés requièrent d'effectuer des purifications fastidieuses et coûteuses afin de réduire les impuretés, notamment les impuretés métalliques du bleu de méthylène. Malgré les étapes de purifications ultérieures, ces différents procédés conduisent inévitablement à un bleu de méthylène comportant de nombreuses impuretés métalliques et également des impuretés organiques, notamment de l'azur B, de l'azur C et de l'azur A.

Le document WO 2006/032879 affirme pouvoir atteindre un taux d'impuretés métalliques représentant 10% du seuil maximal fixé par la pharmacopée européenne, mais d'après les exemples on constate que ce taux n'est pas obtenu simultanément pour tous les métaux et que les résultats des étapes de purification ne sont pas toujours reproductibles. Une analyse détaillée des contenus en métaux de différents bleus de méthylène disponibles commercialement est illustrée dans ce document.

La pharmacopée européenne a été modifiée récemment (avril 2006) dans le sens d'une augmentation des seuils de tolérance des impuretés métalliques car aucun producteur de bleu de méthylène n'était en mesure de produire, et encore moins de produire en quantité industrielle, un bleu de méthylène d'une qualité répondant à ses précédentes exigences.

L'invention est associée à la mise au point d'un procédé de préparation du bleu de méthylène qui donne accès à un bleu de méthylène d'une grande pureté, notamment qui comporte un très faible taux d'impuretés métalliques et organiques, qui soit extrapolable à l'échelle industrielle dans des conditions économiques satisfaisantes, et qui ne soit pas sujet à des variations de qualité. Selon une variante, le procédé est un procédé de purification du bleu de méthylène.

Le procédé est un procédé de préparation de bleu de méthylène répondant à formule (I) ci-dessous :

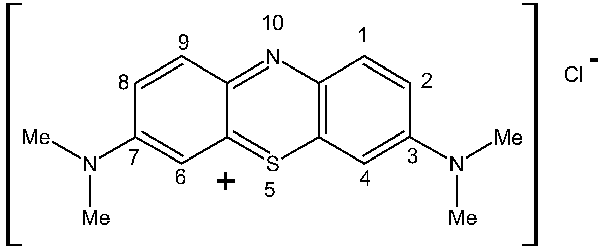

Ce procédé comporte au moins une étape au cours de laquelle un composé de formule (II) : 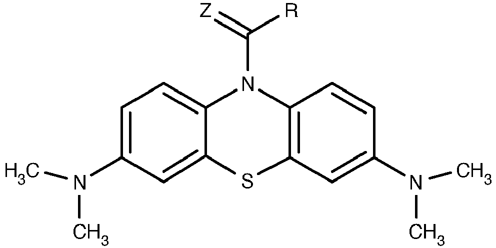 est soumis à une étape de purification dans des conditions permettant de séparer des composés métalliques des composés de formule (II), dans laquelle R représente un groupement choisi parmi :
- un groupement phényle ou benzyle, éventuellement substitués par une ou plusieurs fonctions choisies parmi : un alkyle en C₁-C₄, un atome d'halogène, un halogénoalkyle en C₁-C₄, un groupement nitro,
- un groupement alkyle en C₁-C₈, linéaire, ramifié ou cyclique,
- un groupement alkyl amino en C₁-C₈,
- un groupement alcoxy en C₁-C₈,
- un groupement phényloxy ou benzyloxy éventuellement substitués sur le noyau aromatique par une ou plusieurs fonctions choisies parmi : un alkyle en C₁-C₄, un atome d'halogène, un halogénoalkyle en C₁-C₄, un groupement nitro,
   Z représente un atome choisi parmi O et S.

La purification des composés de formule (II) est faite dans des conditions permettant de séparer les composés métalliques des composés de formule (II) : filtration sur un support susceptible de retenir les composés métalliques, cristallisation dans un solvant approprié ou toute autre méthode connue de l'homme du métier.

Lorsque la purification est faite par filtration sur un support susceptible de retenir les composés métalliques, un tel support peut être choisi parmi : un gel de silice, un gel d'alumine (neutre, basique ou acide), une diatomite éventuellement modifiée, de la célite, une membrane microporeuse, les résines greffées par des groupements capteurs de métaux et les fibres greffées par des groupements capteurs de métaux, tels que des fonctions thiol, acide carboxylique, amine tertiaire, ou tout autre support ayant la propriété de retenir les métaux. Parmi les fibres greffées, on peut citer notamment les produits commercialisés par la société Johnson Matthey sous la marque Smopex ^{®}. Parmi les diatomites, on peut citer les produits commercialisés par la société CECA sous la marque Clarcel ^{®}.

Le composé de formule (II) peut être obtenu à partir du composé de formule (I), par réduction du composé de formule (I) puis par réaction de la fonction amine du cycle phénothiazinium avec un groupement protecteur approprié R-CZ-Y dans lequel R et Z ont la même définition que ci-dessus et Y représente un groupe partant choisi parmi : un atome d'halogène tel que F, Cl, I, Br, un groupement alcoxy en C₁-C₆, un groupement - OCOR (anhydride), un groupement hydroxyle, éventuellement en présence d'un activateur du type dicyclohexylcarbodiimide (DCC). Avantageusement R est choisi parmi un groupement phényle, un groupement toluyl.

Lorsque le composé de formule (II) est obtenu à partir du composé de formule (I), le procédé global est une purification du composé de formule (I). Toutefois, le composé de formule (II) peut être obtenu par d'autres procédés qui n'utilisent pas le produit (I) comme produit de départ.

Certains composés de formule (II) tels que le benzoyl leuco bleu de méthylène sont disponibles commercialement.

Le composé représenté par la formule (I), peut être représenté par plusieurs structures résonnantes équivalentes. A titre d'illustration et de façon non limitative, on fait figurer ci-dessous d'autres structures qui sont équivalentes à celle de la formule (I) :

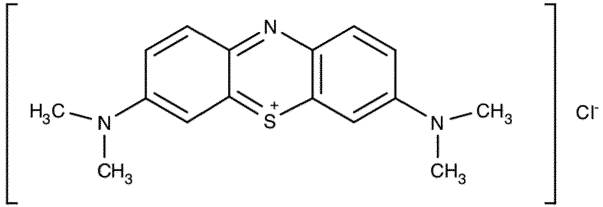

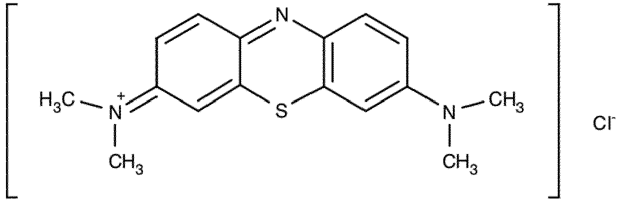

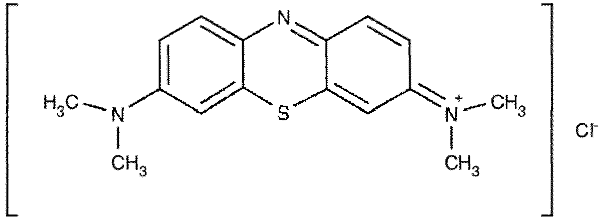

Avantageusement Z représente O.

Selon l'invention le composé de formule (I) est le chlorure de tétraméthylthionine ou bleu de méthylène.

Le procédé de préparation du composé de formule (I), comporte au moins une étape de purification d'un composé de formule (II), en particulier cette purification comporte au moins une étape de filtration d'un composé de formule (II) sur un support susceptible de retenir les composés métalliques, tel qu'un gel de silice, d'alumine (neutre, basique ou acide), une diatomite éventuellement modifiée, une résine fonctionnalisée par des capteurs de métaux, des fibres fonctionnalisées par des capteurs de métaux, de la célite, une membrane microporeuse ou tout autre support capable de retenir les composés métalliques.

De façon plus détaillée, selon cette variante, le composé de formule (II) est mis en solution dans un solvant approprié, on prépare un filtre avec le support de filtration que l'on introduit dans un récipient approprié, tel qu'une colonne de verre, un filtre en verre fritté ou une essoreuse industrielle. Le récipient rempli du support de filtration choisi est humidifié, préférentiellement par le même solvant que celui dans lequel est dissout le composé de formule (II).

La solution contenant le composé de formule (II) est déposée sur le filtre, la solution qui traverse le filtre est récupérée, le filtre est rincé plusieurs fois par un solvant identique ou différent de celui ayant servi à solubiliser le composé de formule (II). Les fractions éluées sont récupérées et éventuellement concentrées.

Parmi les solvants utilisables pour solubiliser les composés de formule (II) on peut citer préférentiellement : les solvants chlorés, comme par exemple le dichlorométhane ou le chloroforme, les alcools tels que isopropanol, éthanol, méthanol ou acétonitrile, acétate d'éthyle, tétrahydrofurane, ou un mélange de ces solvants.

La solution du composé de formule (II) est avantageusement d'une concentration allant de 1 g/l à 10³ g/l. Des concentrations plus faibles conduisent à utiliser des volumes de solvant trop importants avec des conséquences sur la sécurité et la taille du matériel. Des concentrations plus importantes sont difficilement envisageables en raison de la solubilité des produits.

On prévoit d'utiliser environ 0,1 à 10 kg de support de filtration par kg de produit à filtrer. On prévoit avantageusement de rincer le filtre avec 0,1 à 50 l de solvant par kg de produit de formule (II) jusqu'à élution totale du produit de formule (II). Le procédé présente l'avantage de débarrasser le produit de formule (II) de ses impuretés métalliques.

Lorsque l'on choisit de purifier le composé de formule (II) par cristallisation, on choisit avantageusement un solvant parmi : un alcool comme l'éthanol, un solvant chloré comme le chlorure de méthylène.

De façon avantageuse, la fabrication du composé de formule (II) se fait à partir du composé de formule (I) que l'on fait réagir avec un groupement de protection R-CZ-Y dans lequel Y est avantageusement choisi parmi : F, Cl, Br, I, un groupement alcoxy en Ci-Ce, un groupement -OCOR (anhydride), un groupement hydroxyle, éventuellement en présence d'un activateur du type dicyclohexylcarbodiimide (DCC).

La réaction se fait de façon classique en milieu basique ou neutre dans de l'eau ou dans un mélange d'eau et d'un autre solvant tel que par exemple acétonitrile, tétrahydrofurane, dichlorométhane ou tout autre solvant organique approprié.

La réaction est exothermique, et on utilise de préférence des moyens de refroidissement qui permettent de maintenir la température du mélange aux environs de la température ambiante.

Le produit de départ (I) est soit commercial, soit préparé par des méthodes connues, telles que celles décrites dans WO 2006/032879.

D'une façon générale le produit de formule (I) est préparé par des procédés de synthèse qui font appel à l'utilisation de dérivés métalliques que l'on retrouve comme impuretés dans le produit (I), le bleu de méthylène.

Le composé de formule (I) ne peut pas être débarrassé de ses impuretés métalliques et organiques de façon directe, simple et efficace. Les méthodes de l'art antérieur font appel à des recristallisations successives qui n'ont pas des rendements satisfaisants et qui conduisent à des produits dont le taux d'impuretés résiduelles est difficile à contrôler.

En outre le produit de formule (I) a la propriété de chélater les métaux alors que les produits (II) sont non chélatants. Leur purification est donc beaucoup plus efficace que la purification directe du composé de formule (I).

Dans les différentes étapes du procédé, on veille à employer des matériaux non métalliques, des réactifs et des solvants dépourvus de résidus métalliques de façon à ne pas introduire de contamination externe.

Après que le produit de formule (II) ait été purifié, en particulier soumis à une filtration, on procède avantageusement à une étape de déprotection de l'amine du cycle phénothiazine du composé de formule (II). Cette déprotection est faite par tout moyen connu de l'homme du métier, en évitant l'introduction de contaminants métalliques et dans des conditions évitant la dégradation du composé de formule (I). Parmi les moyens utilisables pour la déprotection du groupe R-CZ- on peut citer : les quinones, comme par exemple la 2,3-dichloro-5,6-dicyano-1,4-benzoquinone (DDQ), HNO₃, HClO₄, I₂, HCl, H₂SO₄, H₂O₂, un traitement par des rayonnements ultraviolets. De préférence on utilise une quinone pour cette étape, et de façon très préférentielle la 2,3-dichloro-5,6-dicyano-1,4-benzoquinone. De façon avantageuse, cette réaction de déprotection est faite dans un solvant choisi parmi : l'acétate d'éthyle, l'acétonitrile, le tétrahydrofurane, l'acétone. Le solvant préféré pour cette étape est l'acétonitrile.

Des conditions de déprotection avantageuses prévoient l'emploi de 0,80 à 1,1 équivalents molaires de DDQ par rapport au composé (II), encore plus avantageusement de 0,85 à 1,05 équivalents molaires de DDQ par rapport au composé (II), avantageusement de 0,90 à 1 équivalent molaire. Préférentiellement, cette déprotection se fait à une température comprise entre -40°C et -5°C. Bien que non totalement exclues, une température plus basse aurait l'inconvénient d'allonger les durées de réaction, une température plus élevée pourrait conduire à la formation de produits secondaires.

En fonction du moyen de déprotection employé on peut être amené à faire un échange d'ions pour aboutir au composé de formule (I) comportant l'anion X⁻ souhaité. Cet échange d'ion est fait par traitement avec HCl, avantageusement dans l'acétate d'éthyle. D'autres solvants pourraient être utilisés, mais certains sont susceptibles de conduire à la formation de produits secondaires.

Les conditions de déprotection des composés de formule (II) exposées ci-dessus sont particulièrement avantageuses en ce qu'elles permettent d'aboutir à un composé de formule (I) sans introduire au cours de cette étape d'impuretés métalliques ou former d'impuretés organiques. Selon une variante de l'invention, on peut prévoir de purifier le composé de formule (II) par d'autres moyens que la filtration sur un support susceptible de retenir les métaux, comme par exemple par cristallisation dans un solvant approprié. Selon cette variante, on déprotège ensuite le composé de formule (II) à l'aide de tout moyen de déprotection n'impliquant pas l'utilisation de composés métalliques, en particulier à l'aide d'une quinone, en particulier le DDQ, de préférence dans les conditions exposées ci-dessus.

Le Bleu de méthylène peut être obtenu par un procédé de préparation caractérisé en ce qu'il comporte au moins une étape de déprotection du groupe R-CZ- de l'amine du cycle phénothiazine du composé de formule (II) à l'aide de moyens de déprotection n'impliquant pas l'utilisation de composés métalliques.

Par « moyens de déprotection n'impliquant pas l'utilisation de composés métalliques », on entend l'emploi de réactifs non métalliques, de solvants ne comportant pas de résidus métalliques (de préférence < 0,01 ppm), dans des réacteurs ne comportant pas de parties métalliques, comme des réacteurs émaillés par exemple.

Parmi les moyens utilisables pour la déprotection du groupe R-CZ- on peut citer : les quinones, comme par exemple la 2,3-dichloro-5,6-dicyano-1,4-benzoquinone (DDQ), HNO₃, HClO₄, I₂, HCl, H₂SO₄, H₂O₂, un traitement par des rayonnements ultraviolets. De préférence on utilise une quinone pour cette étape, et de façon très préférentielle la 2,3-dichloro-5,6-dicyano-1,4-benzoquinone. Avantageusement on emploie les conditions de mise en oeuvre du DDQ qui ont été décrites ci-dessus.

La méthode de déprotection du composé (II) en composé (I) permet d'aboutir à un composé (I) qui ne comporte pas d'impuretés métalliques additionnelles par rapport au produit (II). En outre, ces conditions de déprotection évitent la formation de produits de dégradation organique. En effet, le composé de formule (I) est d'une stabilité limitée et l'utilisation de certaines conditions de traitement conduit à des dégradations, par exemple du bleu de méthylène en Azur A, B et C, qui sont ensuite difficilement séparables.

Le procédé de l'invention permet d'avoir accès à un composé de formule (I) dépourvu de contaminants métalliques et d'une haute pureté chimique, de façon fiable, reproductible et applicable à l'échelle industrielle. Ces qualités sont essentielles pour pouvoir fournir un produit de formule (I) de qualité pharmaceutique.

Notamment le procédé de préparation ou de purification décrit ci-dessus permet d'obtenir en quantités industrielles et de façon reproductible un bleu de méthylène ou chlorure de tétraméthylthionine qui comprend 0,02 µg/g ou moins de cadmium par g de bleu de méthylène. Le procédé décrit ci-dessus donne accès à un bleu de méthylène ou chlorure de tétraméthylthionine ayant un taux de pureté supérieur à 97%, préférentiellement supérieur à 98%, encore mieux supérieur à 99%, mesurée en CLHP (chromatographie liquide haute performance) dans les conditions de la pharmacopée européenne 5.4 (édition d'avril 2006) et comprenant moins de 4,5 µg/g d'aluminium, avantageusement moins de 3 µg/g d'aluminium, encore plus avantageusement moins de 2,5 µg/g d'aluminium par g de bleu de méthylène.

Le procédé décrit ci-dessus donne accès à un bleu de méthylène ou chlorure de tétraméthylthionine ayant un taux de pureté supérieur à 97%, préférentiellement supérieur à 98%, encore mieux supérieur à 99%, mesurée en CLHP dans les conditions de la pharmacopée européenne 5.4 (édition d'avril 2006) et comprenant moins de 0,5 µg/g d'étain par g de bleu de méthylène.

Le procédé décrit ci-dessus donne accès à un bleu de méthylène ou chlorure de tétraméthylthionine ayant un taux de pureté supérieur à 97%, préférentiellement supérieur à 98%, encore mieux supérieur à 99%, mesurée en CLHP dans les conditions de la pharmacopée européenne 5.4 (édition d'avril 2006) et comprenant moins de 0,95µg/g de chrome, avantageusement moins de 0,90µg/g, encore mieux, moins de 0,80µg/g par g de bleu de méthylène.

Le procédé décrit ci-dessus est le seul à donner accès, en quantité industrielle, à un bleu de méthylène ou chlorure de tétraméthylthionine comprenant moins de 3% d'impuretés, préférentiellement moins de 2%, encore mieux moins de 1%, mesuré en CLHP dans les conditions de la pharmacopée européenne 5.4 (édition d'avril 2006) et un taux d'impuretés métalliques inférieur à 20 µg/g, avantageusement inférieur à 15 µg/g, encore plus avantageusement inférieur à 10 µg/g.

Le bleu de méthylène est utilisé depuis des décennies dans le traitement de diverses infections. Il est utilisé comme agent antiseptique, anti infectieux, comme antidote et comme agent de diagnostique. De façon récente son activité antivirale a été mise en évidence, et il pourrait être utilisé dans la préparation d'un médicament destiné à lutter contre une pathologie telle qu'une infection, notamment un choc septique, la présence de contaminants pathogéniques dans le sang ou le plasma, une réaction hémodynamique excessive, une infection par le HIV, le virus West Nile, le virus de l'hépatite C, la maladie d'Alzheimer, la malaria, le cancer du sein, les troubles maniaco-dépressifs.

Enfin il pourrait également être utilisé en cosmétique ou pour des produits destinés à une application ophtalmique.

Pour toutes ces applications thérapeutiques, et en particulier dans le contexte de la prévention et du traitement de la maladie d'Alzheimer, il est nécessaire de disposer d'un bleu de méthylène ayant un degré de pureté élevé et en particulier comportant très peu d'impuretés métalliques.

Un médicament comprenant un bleu de méthylène de l'invention, dans un support pharmaceutiquement acceptable, constitue un autre objet de l'invention.

Le support et les quantités de bleu de méthylène à administrer sont bien connus de l'homme du métier.

La demande décrit un procédé de préparation d'un médicament comportant un composé de formule (I), caractérisé en ce que ce procédé comporte au moins une étape de procédé telle que décrite ci-dessus, en particulier une étape de purification du composé de formule (I) et/ou une étape de déprotection du composé (II) en (I).

### PARTIE EXPERIMENTALE

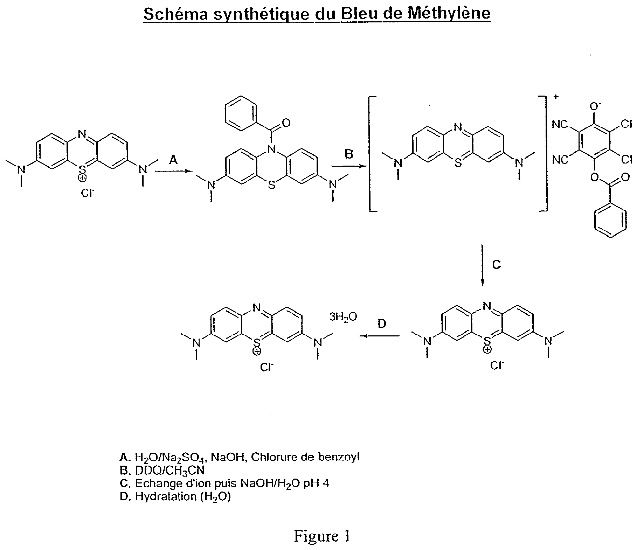

Un bleu de méthylène commercial est purifié conformément au procédé de la figure 1.

### 1- Synthèse du Benzoyl leuco bleu de Méthylène (Etape A)

Dans un réacteur double enveloppe de 120 L muni d'une agitation et sous azote on introduit :
- 80 L d'eau distillée,
- 4,2 kg (10,7 moles) de bleu de méthylène commercialisé par la société LEANCARE LTD sous la référence CI 52015, comprenant d'importantes quantités d'impuretés métalliques (Al, Fe, Cu, Cr).

On laisse 15 min sous agitation puis on ajoute 6,9 kg de sodium hydrosulfite Na₂S₂O₄ en solution aqueuse à 85%. La coloration vire du bleu vers le beige. On laisse 45 min supplémentaires sous agitation, puis on ajoute 2,69 kg de soude sous forme de pastilles. La température de la réaction est maintenue entre 18 et 20°C. La durée d'addition est de 30 min et on laisse sous agitation 30 min supplémentaires. Ensuite on ajoute goutte-à-goutte 7,90 L de chlorure de benzoyle. Le mélange réactionnel vire vers une coloration vert-beige. La durée d'addition est de 2h et ensuite on laisse sous agitation pendant 20 h.

### Traitement :

Après arrêt de l'agitation on laisse décanter 15 min et on aspire le surnageant. On ajoute 80 L d'eau (25 volumes) et après agitation et décantation on aspire de nouveau le surnageant. On ajoute 24 L d'EtOH et après une agitation d'environ 5 min on ajoute 16 L d'eau. Après avoir agité pendant 15 min, le mélange est filtré sur recette. Cette opération est répétée 3 fois. Après séchage on obtient 2,9 kg (Rdt : 66%) de benzoyl leuco bleu de méthylène.

### 2- Purification

On utilise 4,25 kg de benzoyl leuco bleu de méthylène issu de la première étape, solubilisé dans 30 L de CH₂Cl₂. On filtre sur 3 parts de silice (Merck Gerudan Si60) (11,5 kg) et 0,5kg de sable de Fontainebleau on rince avec 30 litres de CH₂Cl₂. On élimine CH₂Cl₂ par évaporation sous vide. On ajoute 6 L d'éthanol. On laisse sous agitation à froid puis on filtre sur recette. On sèche sous vide. On obtient 3,4 kg de benzoyl leuco bleu de méthylène purifié (Rdt : 80%).
Pureté : +99 % CLHP
Métaux : le contenu en métaux (en µg/g) est donné pour 3 essais dans le tableau 1.

**Tableau 1**

| Essai | Essai 1 | Essai 2 | Essai 3 |
|---|---|---|---|
| Al | **0,5** | **0,5** | **0,1** |
| Cu | **0** | **0** | **0,4** |
| Fe | **0** | **0** | **0,1** |
| Zn | **0,9** | **0,7** | **0,5** |
| Ni | **0,1** | **0,1** | **0,1** |
| Cr | **0,3** | **0,3** | **0,03** |
| Mo | **0,1** | **0,1** | **0,1** |
| Mn | **0,02** | **0** | **0** |
| Sn | **0,5** | **0,4** | **0,5** |
| Pb | **5** | **3,2** | **2,4** |
| Cd | **0,2** | **0,2** | **0,07** |

### 3- Débenzoylation

Dans un réacteur double enveloppe émaillé de 100 l à température ambiante, on introduit :
- 45 L d'acétonitrile (ACN)
- 1,6 kg du benzoyl leuco bleu de méthylène issu de la seconde étape et on place sous agitation. On laisse agiter 30 min à température ambiante puis on baisse la température à -18°C. On ajoute en une portion 950 g de DDQ solubilisés dans 4 L d'ACN. On laisse agiter 2h à -18°C. On filtre. On obtient un complexe du 3,7-bis(diméthyl amino)phénothiazine avec le DDQ qui est utilisé directement dans l'étape suivante.

### 4- Salification

Dans le réacteur double enveloppe émaillé, on réintroduit le gâteau issu de la troisième étape en plusieurs morceaux. On ajoute 4 L d'AcOEt. On laisse tourner pendant 30 min à température ambiante. On baisse la température à -18°C. On ajoute 2,5 kg d'HCl dans 16 L d'AcOEt (solution 4N) . On agite 2h à -18°C. On filtre puis on réintroduit le gâteau dans le réacteur. On ajoute 30 L d'AcOEt à -18°C et on filtre à nouveau.

### 5- Neutralisation

On ajoute 30 L d'acétone puis une solution de 200 g de NaOH solubilisés dans 500 ml d'eau. On filtre.On introduit le produit issu de la quatrième étape dans le réacteur avec 30 L d'acétone. On agite le milieu pendant 1h à température ambiante. Le pH est de 4,0. On filtre. On laisse sous vide sur la recette.

### 6- Purification et hydratation

Dans un réacteur émaillé de 40 L sous N₂, à température ambiante, on a introduit 1,9 kg du produit de la cinquième étape et 30 L du mélange 50/50 CH₂Cl₂/EtOH. On chauffe au reflux (43°C). On filtre à chaud avec un filtre microfibre (Whatman GF/D). Cette opération est effectuée 2 fois. On nettoie le réacteur à l'eau déminéralisée. On réintroduit le filtrat dans le réacteur. On distille sous vide à 28°C 24 litres de solvant (3h). Le milieu est remis dans le réacteur. On ajoute 1 L d'eau microfiltrée. On refroidit à -18°C. On ajoute 40 L d'AcOEt et on laisse la nuit sous agitation à froid. On filtre. On empatte avec 10 L d'AcOEt. On obtient 1,4 kg de bleu de méthylène purifié sous la forme trihydratée.

Les impuretés métalliques sont analysées et rapportées dans le tableau 2.

**Tableau 2**

| élément | Quantité (µg/g) |
|---|---|
| Al | **1,3** |
| Cu | **0,5** |
| Fe | **1,9** |
| Zn | **1,7** |
| Ni | **0,5** |
| Cr | **0,8** |
| Mo | **0,2** |
| Mn | **0,08** |
| Sn | **0,4** |
| Pb | **0,1** |
| Cd | **0,04** |

## Revendications

1. Bleu de méthylène comprenant moins de 3% d'impuretés, mesuré en CLHP dans les conditions de la pharmacopée européenne 5.4, et un taux de métaux inférieur à 20 µg/g.

2. Bleu de méthylène selon la revendication 1 comprenant moins de 3% d'impuretés, mesuré en CLHP dans les conditions de la pharmacopée européenne 5.4, et un taux de métaux inférieur à 15 µg/g.

3. Bleu de méthylène selon la revendication 2 comprenant moins de 3% d'impuretés, mesuré en CLHP dans les conditions de la pharmacopée européenne 5.4, et un taux de métaux inférieur à 10 µg/g.

4. Médicament comprenant un bleu de méthylène selon l'une quelconque des revendications 1 à 3 dans un support pharmaceutiquement acceptable.

5. Utilisation d'un bleu de méthylène selon l'une quelconque des revendications 1 à 3 pour la préparation d'un médicament destiné à la prévention ou au traitement d'une pathologie sélectionnée parmi :
une infection, un choc septique, la présence de contaminants pathogéniques dans le sang ou le plasma, une réaction hémodynamique excessive, une infection par le HIV, le virus West Nile, le virus de l'hépatite C, la maladie d'Alzheimer, la malaria, le cancer du sein, les troubles maniaco-dépressifs.

## Patentansprüche

1. Methylenblau mit weniger als 3% Verunreinigungen, gemessen durch HPLC unter den Bedingungen des Europäischen Arzneibuchs 5.4, und einem Gehalt an Metallen von weniger als 20 µg/g.

2. Methylenblau nach Anspruch 1, mit weniger als 3% Verunreinigungen, gemessen durch HPLC unter den Bedingungen des Europäischen Arzneibuchs 5.4, und einem Gehalt an Metallen von weniger als 15 µg/g.

3. Methylenblau nach Anspruch 2, mit weniger als 3% Verunreinigungen, gemessen durch HPLC unter den Bedingungen des Europäischen Arzneibuchs 5.4, und einem Gehalt an Metallen von weniger als 10 µg/g.

4. Medikament, das Methylenblau gemäß einem der Ansprüche 1 bis 3 in einem pharmazeutisch unbedenklichen Träger umfasst.

5. Verwendung von Methylenblau gemäß einem der Ansprüche 1 bis 3 für die Herstellung eines Medikaments zur Verhütung oder Bekämpfung einer Pathologie aus einer Infektion, eines septischen Schocks, des Vorliegens von pathogenen Verunreinigungen im Blut oder Plasma, einer übermäßigen hämodynamischen Reaktion, einer Infektion mit HIV, West-Nile-Virus oder Hepatitis-C-Virus, Alzheimer-Krankheit, Malaria, Krebs oder manisch-depressiven Störungen, ausgewält.

## Claims

1. Methylene blue comprising less than 3% of impurities, measured by HPLC under the conditions of the European Pharmacopeia 5.4, and a level of metals of less than 20 µg/g.

2. Methylene blue according to claim 1, comprising less than 3% of impurities, measured by HPLC under the conditions of the European Pharmacopeia 5.4, and a level of metals less than 15 µg/g.

3. Methylene blue according to claim 2, comprising less than 3% of impurities, measured by HPLC under the conditions of the European Pharmacopeia 5.4, and a level of metals less than 10 µg/g.

4. A medicament comprising a methylene blue as claimed in any one of claims 1 to 3, in a pharmaceutically acceptable carrier.

5. The use of a methylene blue as claimed in any one of claims 1 to 3, for the preparation of a medicament for preventing or treating a pathological condition selected from:
an infection, a septic shock, the presence of pathogenic contaminants in the blood or the plasma, an exaggerated hemodynamic reaction, an infection with HIV, West Nile virus or the hepatitis C virus, Alzheimer's disease, malaria, breast cancer and manic depressive disorders.
